# EUROPEAN PATENT APPLICATION

(11) **EP 4 144 363 A1**
(43) Date of publication of application: **08.03.2023**
(21) Application number: 20933171.9
(22) Date of filing: 04.12.2020
(51) Int. Cl.: A61K 38/47, A61K 31/704, A61K 45/06, A61P 31/14, A61P 11/00, A61P 1/00, A23L 33/10, A23L 33/17

(54) **DRUG, FOOD AND APPLICATION OF ANTI-CORONAVIRUS INFECTION**

(30) Priority: 27.04.2020 CN 202010341811
(71) Applicant: Guangzhou Century Clinical Research Co., Ltd, Guangzhou, Guangdong 510530 (CN); Guangzhou Xin-Chuangyi Biopharmaceutical Co., Ltd, Huangpu District Guangzhou, Guangdong 510535 (CN); Guangzhou Xin Chuang Yi Co., Ltd, Guangzhou, Guangdong 510535 (CN); Xiangbei Welman Pharmaceutical Co., Ltd, Hunan 410331 (CN); Nanjing Kangfushun Pharmaceutical Co., Ltd, Nanjing, Jiangsu 210046 (CN)
(72) Inventor: SUN, Mingjie, Guangzhou, Guangdong 510620 (CN); SUN, Tianyu, Guangzhou, Guangdong 510620 (CN); LI, Changqing, Guangzhou, Guangdong 510620 (CN)
(74) Representative: Ladendorf, Oliver
(86) International application number: PCT/CN2020/133898
(87) International publication number: WO 2021/218154

(57) **Abstract**

A drug, food and application of an anti-coronavirus infection, the drug or food containing lysozyme or a combination that comprises lysozyme; the lysozyme or a combination thereof can be used to prepare a drug for anti coronavirus infection and preventing or treating coronavirus-related diseases or symptoms, or food for assisting in anti coronavirus infection and assisting in preventing or treating coronavirus-related diseases or symptoms. Lysozyme or a combination thereof can effectively inhibit the cell damage and inflammatory reaction caused by coronavirus, relieve symptoms of the digestive system, and improve airway obstruction, decreased expiratory volume and intestinal barrier function. The present disclosure has a good preventive and therapeutic effect on coronavirus infection or related diseases or symptoms thereof; in addition, the present application can accelerate the elimination of the virus in vivo, accelerate the speed of coronavirus nucleic acid tests in vivo turning negative, shorten the course of a patient's illness, effectively prevent and reduce the spread of coronavirus, and also has important social benefits and good application prospects.

## Description

### TECHNICAL FIELD

The present disclosure relates to the field of medicine, and more particularly, to a drug, food and application for anti novel coronavirus infection and preventing and treating the novel coronavirus-related disease or symptom.

### BACKGROUND

The World Health Organization announced in March 2020 that the outbreak of COVID-19 caused by novel coronavirus infection has become a global pandemic, and it is still a huge burden and threat to the global public health. Up to now, the number of infected people in the world has not yet reached an inflection point, and after the spread of the epidemic in some areas has been successfully controlled through various measures, second and third outbreaks have occurred, and the global COVID-19 epidemic continues to spread. According to data from the World Health Organization, at present, the cumulative number of confirmed cases in the world has exceeded 51 million, and has caused more than 1.27 million deaths.

Novel coronavirus is a new virus belonging to the coronaviridae family, which has some similarities with SARS virus, but also has obvious differences, especially in virus gene sequence, asymptomatic carrier, clinical symptom, histopathology, etc. Pathological observation shows that the degree of pulmonary fibrosis of patients suffering from COVID-19 is lower than that of patients suffering from SARS, but degrees of pulmonary hyperplasia and obstruction of the patients suffering from COVID-19 are higher than those of the patients suffering from SARS.

Compared with the SARS virus, the novel coronavirus has a higher infection rate, a longer incubation period, a stronger concealment, a shorter continuous interval and a longer virus detoxification time. Novel coronavirus, through its spike protein (S protein), binds to human angiotensin converting enzyme 2 (ACE2) and mediates virus invasion into host cells. The binding affinity of the novel coronavirus with ACE2 is very high, which is 10 times to 20 times that of the SARS virus. Although the fatality rate of the novel coronavirus is lower than that of the SARS virus, the number of deaths caused by COVID-19 in the world is four orders of magnitude higher than that of SARS in 2003 due to the scale of the epidemic. In view of the complexity of the COVID-19 epidemic, some scholars believe that it may take more than five years for the global economy to recover, and even believe that the novel coronavirus may coexist with human beings for a long time.

Fever and respiratory symptoms are main manifestations of the COVID-19. With the deepening research on the novel coronavirus, researchers found that except for lungs which were target organs of the novel coronavirus, the novel coronavirus also attacked immune system, urinary system, cardiovascular system, digestive system, and even nervous system of a human body. For example, after reviewing the cases, the researchers found that 12% to 61% of patients had the digestive symptoms. Symptoms of some patients suffering COVID-19 firstly appear in the digestive system rather than the respiratory system or fever, and even 10% patients only show the symptoms in the digestive system in the whole course of the disease. Symptoms of some children and newborns are mainly vomiting and diarrhea.

Although some antiviral drugs, such as interferon, oseltamivir, lopinavir, ritonavir, farpiravir, ribavirin, remdesivir, chloroquine, and arbidol, have been approved or recommended urgently, most of existing drugs have adverse reactions in different degrees, especially for asymptomatic infected patients or mild symptom patients, so that the clinical benefit-risk ratios of the drugs are very low. In addition, the patients' novel coronavirus nucleic acid detection turned negative and then became positive again from time to time. It has also been reported that after the virus nucleic acid detection of respiratory tract samples of some patients turned negative, the stool samples were still positive for a long time. It is indicated that current antiviral drugs cannot control novel coronavirus infection in the digestive system well. The persistent infection of novel coronavirus is an important factor hindering the recovery of the patients. For the asymptomatic infected patients or the patients with mild clinical symptoms in convalescence of COVID-19, if the virus cannot turn negative, continuous centralized isolation and medical observation are needed, thus seriously affecting normal work and life. Rapid elimination of residual virus in the infected patients is a key to turn negative in the novel coronavirus nucleic acid test.

If a drug that is basically harmless to a body, and can inhibit the replication and spread of the novel coronavirus, improve the virus clearance efficiency in vivo and relieve symptoms of novel coronavirus infection can be provided, it will undoubtedly have great social benefits.

### SUMMARY

One of the objects of the present disclosure is to provide an application of lysozyme in preparation of a drug or a food. In order to achieve this object, the technical solutions used in the present disclosure are as follows.

An application of lysozyme or a combination containing lysozyme in preparation of a drug for anti novel coronavirus infection is provided.

An application of lysozyme or a combination containing lysozyme in preparation of a food for assisting in anti novel coronavirus infection is provided.

An application of lysozyme or a combination containing lysozyme in preparation of a drug for preventing or treating novel coronavirus infection-related disease or symptom is provided.

An application of lysozyme or a combination containing lysozyme in preparation of a food for assisting in preventing or treating novel coronavirus infection-related disease or symptom is provided.

In some examples, the novel coronavirus infection comprises novel coronavirus infection of at least one selected from the group consisting of respiratory system, digestive system, urinary system, reproductive system and cardiovascular system, such as lung, upper respiratory tract, lower respiratory tract, stomach, intestine, liver, kidney, testis, placenta, heart and other tissues or organs. The novel coronavirus infection is preferably the novel coronavirus infection of the respiratory system and/or the digestive system, such as lung infection and/or intestine infection.

In some examples, the novel coronavirus infection refers to being positive in a novel coronavirus nucleic acid detection.

In some examples, the novel coronavirus nucleic acid detection may be performed based on a clinical specimen; and the clinical specimen comprises a respiratory tract specimen and/or a digestive tract specimen. In some examples, the respiratory tract specimen may be an upper respiratory tract specimen or a lower respiratory tract specimen. In some examples, the upper respiratory tract specimen may be an oropharyngeal swab and a nasopharyngeal swab; and in some examples, the lower respiratory tract specimen may be a respiratory tract aspirate, a bronchial lavage fluid, an alveolar lavage fluid and a deep expectoration fluid. In some examples, the digestive tract specimen may be collected from esophagus, stomach, small intestine (such as duodenum and ileum), large intestine (such as colon and rectum), anal canal or feces; and specifically, the digestive tract specimen may be, for example, an anal swab and a rectal swab. In some examples, only the digestive tract specimen is positive in the novel coronavirus nucleic acid detection; in other examples, both the digestive tract specimen and the respiratory tract specimen are positive in the novel coronavirus nucleic acid detection.

In some examples, the novel coronavirus infection-related disease or symptom refers to a clinical disease or symptom combined on the basis of the novel coronavirus infection. In some examples, these clinical diseases or symptoms are caused by the novel coronavirus infection.

In some examples, the novel coronavirus infection-related disease or symptom is at least one selected from the group consisting of diseases or symptoms of immune system, diseases or symptoms of respiratory system, diseases or symptoms of digestive system, diseases or symptoms of urinary system, diseases or symptoms of reproductive system, diseases or symptoms of cardiovascular system, and diseases or symptoms of nervous system. In some examples, the novel coronavirus infection-related disease or symptom comprises more than one disease or symptom, such as the disease or symptom of the respiratory system and the disease or symptom of the digestive system.

In some examples, the disease or symptom of the immune system is selected from fever or cytokine storm syndrome.

In some examples, the disease or symptom of the digestive system is selected from intestinal barrier dysfunction or digestive dysfunction. In some examples, the disease or symptom of the digestive system may be at least one selected from the group consisting of diarrhea, nausea, vomiting, anorexia, abdominal pain, gastrointestinal bleeding, constipation and flatulence.

In some examples, the disease or symptom of the respiratory system is selected from increased airway resistance, decreased expiratory volume, pneumonia or acute respiratory distress syndrome.

In some examples, the disease or symptom of the respiratory system is COVID-19 novel coronavirus pneumonia, and the COVID-19 novel coronavirus pneumonia refers to COVID-19 novel coronavirus pneumonia confirmed clinically.

In some examples, the combination containing lysozyme further comprises glycyrrhizic acid or a salt of the glycyrrhizic acid, such as monoammonium glycyrrhizinate and/or dipotassium glycyrrhizinate.

In some examples, a weight ratio of the lysozyme to the glycyrrhizic acid or the salt of the glycyrrhizic acid in the combination is 100: 1 to 1: 100, preferably 100: 1 to 2: 1, more preferably 100: 1 to 5: 1, and further preferably 100: 1 to 10: 1. In some preferred solutions, the lysozyme and the glycyrrhizic acid or the salt of the glycyrrhizic acid may have a significant synergistic effect.

The lysozyme or the combination containing lysozyme provided by the present disclosure may also be combined with other ingredients capable of being used for preventing or treating the novel coronavirus infection-related disease or symptom, by combined medication or being made into a compound preparation, and these ingredients may be active ingredients with a potential of anti the novel coronavirus infection or active ingredients capable of symptomatically treating the disease or symptom.

In some examples, the other ingredients capable of being used for preventing or treating the novel coronavirus infection-related disease or symptom may comprise other ingredients capable of being used for preventing or treating the COVID-19 novel coronavirus pneumonia, also may comprise other ingredients capable of being used for preventing or treating the disease or symptom of the digestive system.

In some examples, the other ingredients capable of being used for preventing or treating the COVID-19 novel coronavirus pneumonia are at least one selected from the group consisting of interferon, oseltamivir or salt thereof, lopinavir, ritonavir, favipiravir, ribavirin, remdesivir, chloroquine or salt thereof, hydroxychloroquine or salt thereof, arbidol or salt thereof, interleukin inhibitors, tumor necrosis factor inhibitors, janus kinase inhibitors, glucocorticoid, protease inhibitors, and intestinal microecological regulators. In some examples, the protease inhibitor may be selected from ulinastatin, sivelestat, nafamostat, or tranexamic acid. In some examples, the interleukin inhibitor may be selected from tocilizumab. In some examples, the tumor necrosis factor inhibitor may be selected from adalimumab, infliximab, or etanercept. In some examples, the janus kinase inhibitor may be selected from tofacitinib or baricitinib. In some examples, the intestinal microecological regulator may be selected from prebiotics or probiotics.

In some examples, the other ingredients capable of being used for preventing or treating the disease or symptom of the digestive system are at least one selected from the group consisting of intestinal microecological regulators, gastrointestinal mucosal protective agents, antiemetic, antidiarrheal, gastrointestinal excitomotors, antispasmodic, anti-ulcer agents, and digestant.

In some examples, the lysozyme in the combination may be combined with at least one selected from the group consisting of glycyrrhizic acid or the salt of the glycyrrhizic acid, the other ingredients capable of being used for preventing or treating the COVID-19 novel coronavirus, and the other ingredients capable of being used for preventing or treating the disease or symptom of digestive system. In some examples, the lysozyme and the glycyrrhizic acid or the other ingredients may be combined by respectively existing in different drugs (such as combined packaging and combined medication); and in some examples, the lysozyme and the glycyrrhizic acid or the other ingredients may be combined by co-existing in the same drug (such as a compound preparation).

In some examples, dosage forms of the drug comprise an oral dosage form, an injection dosage form, an inhalation dosage form, and a cavity dosage form.

In some examples, release form of the drug comprise normal release, delayed release, sustained release, or controlled release.

In some examples, the drug further comprises a pharmaceutically acceptable excipient.

In some examples, the food is a dietary supplement or a food for special medical use.

In some examples, the food further comprises an additive suitable for food. In some examples, the additive may be a preservative, a colorant, a flavoring agent, a spice, an anti staling agent, an antioxidant, an emulsifier, a thickening agent, a carbohydrate, a fat, a vitamin, an amino acid, a trace element, a protein, and the like.

In some examples, the lysozyme and the glycyrrhizic acid or the salt of the glycyrrhizic acid in the combination may be combined in various ways. The lysozyme and the glycyrrhizic acid or the salt of the glycyrrhizic acid may be combined by respectively existing in different foods, such as combined packaging and combined use; or may be combined by co-existing in the same food, such as a compound food.

The second object of the present disclosure is to provide a drug or a food related to novel coronavirus infection, and in order to achieve the object, the technical solutions used in the present disclosure are as follows.

A drug, which comprises lysozyme, and the drug is used for anti novel coronavirus infection or preventing/treating novel coronavirus infection-related disease or symptom.

A food, which comprises lysozyme, and the food is used for assisting in anti novel coronavirus infection and assisting in preventing or treating novel coronavirus infection-related disease or symptom.

In some examples, the novel coronavirus infection is as described in the part of the first object of the present disclosure - "an application of lysozyme in preparation of a drug or a food".

In some examples, the novel coronavirus infection-related disease or symptom is as described in the part of the first object of the present disclosure - "an application of lysozyme in preparation of a drug or a food".

In some examples, the drug or the food further comprises glycyrrhizic acid or a salt of the glycyrrhizic acid, such as monoammonium glycyrrhizinate and/or dipotassium glycyrrhizinate.

In some examples, a weight ratio of the lysozyme to the glycyrrhizic acid or the salt of the glycyrrhizic acid in the drug or the food is 100: 1 to 1: 100, preferably 100: 1 to 2: 1, more preferably 100: 1 to 5: 1, and further preferably 100: 1 to 10: 1. In some preferred solutions, the lysozyme and the glycyrrhizic acid or the salt of the glycyrrhizic acid may have a significant synergistic effect.

In some examples, the drug further comprises other ingredients capable of being used for preventing or treating the novel coronavirus infection-related disease or symptom. Specific ingredients are as described in the part of the first object of the present disclosure - "an application of lysozyme in preparation of a drug or a food".

In some examples, the lysozyme and the glycyrrhizic acid or the salt of the glycyrrhizic acid, or the other ingredients in the drug, or the lysozyme and the glycyrrhizic acid or the salt of the glycyrrhizic acid in the food, may both be combined in various ways. Specific combination ways are as described in the part of the first object of the present disclosure - "an application of lysozyme in preparation of a drug or a food".

In some examples, dosage forms of the drug may be various dosage forms suitable for medication, such as an oral dosage form, an injection dosage form, an inhalation dosage form, and a cavity dosage form.

In some examples, the drug may be preparations of different release forms, such as a normal release preparation, a delayed release preparation, a sustained release preparation, or a controlled release preparation.

In some examples, the drug is an enteric preparation, and the preparation may control the release of active ingredients mainly in the intestine. The enteric preparation may be divided according to physical forms, such as an enteric tablet, an enteric capsule, an enteric granule and an enteric pellet; and the enteric preparation may be divided according to specific release sites of the active ingredients, such as a duodenum enteric preparation, a jejunum enteric preparation, an ileum enteric preparation, a cecum enteric preparation, a colon enteric preparation or a rectum enteric preparation.

In some examples, the drug further comprises a pharmaceutically applicable excipient.

In some examples, the food is a dietary supplement or a food for special medical use.

In some examples, the food further comprises an additive suitable for food. In some examples, the additive may be a preservative, a colorant, a flavoring agent, a spice, an anti staling agent, an antioxidant, an emulsifier, a thickening agent, a carbohydrate, a fat, a vitamin, an amino acid, a trace element, a protein, and the like.

The third object of the present disclosure is to provide a method, comprising a method for anti novel coronavirus infection, a method for assisting in anti novel coronavirus, a method for preventing or treating novel coronavirus infection-related disease or symptom, and a method for assisting in preventing or treating novel coronavirus infection-related disease or symptom.

In order to achieve the object, the technical solutions used in the present disclosure are as follows.

A method for resisting novel coronavirus infection and preventing/treating novel coronavirus infection-related disease or symptom, comprises administering a drug to a subject in need thereof, wherein the drug comprises an effective amount of lysozyme, or the drug comprises an effective amount of a combination comprising lysozyme.

A method for assisting in resisting novel coronavirus infection or assisting in preventing/treating novel coronavirus infection-related disease or symptom, comprises feeding a food to a subject in need thereof, wherein the food comprises an effective amount of lysozyme, or the food comprises an effective amount of a combination comprising lysozyme.

In some examples, the novel coronavirus infection is as described in the part of the first object of the present disclosure - "an application of lysozyme in preparation of a drug or a food".

In some examples, the novel coronavirus infection-related disease or symptom is as described in the part of the first object of the present disclosure - "an application of lysozyme in preparation of a drug or a food".

In some examples, the drug or the food further comprises glycyrrhizic acid or a salt of the glycyrrhizic acid, such as monoammonium glycyrrhizinate and/or dipotassium glycyrrhizinate.

In some examples, a weight ratio of the lysozyme to the glycyrrhizic acid or the salt of the glycyrrhizic acid in the drug or the food is 100: 1 to 1: 100, preferably 100: 1 to 2: 1, more preferably 100: 1 to 5: 1, and further preferably 100: 1 to 10: 1. In some preferred solutions, the lysozyme and the glycyrrhizic acid or the salt of the glycyrrhizic acid may have a significant synergistic effect.

In some examples, the drug further comprises other ingredients capable of being used for preventing or treating the novel coronavirus infection-related disease or symptom. Specific ingredients are as described in the part of the first object of the present disclosure - "an application of lysozyme in preparation of a drug or a food".

In some examples, the subject is positive in the novel coronavirus nucleic acid detection, or is clinically confirmed to suffer from the COVID-19 novel coronavirus pneumonia.

In some examples, the lysozyme and the glycyrrhizic acid or the salt of the glycyrrhizic acid, or the other ingredients in the drug or the food, or the lysozyme and the glycyrrhizic acid or the salt of the glycyrrhizic acid in the food may both be combined in various ways. Specific combination ways are as described in the part of the first object of the present disclosure - "an application of lysozyme in preparation of a drug or a food".

In some examples, administration methods of the drug may be various methods, such as oral administration, injection, inhalation, and cavity administration.

In some examples, the drug may be preparations of different release forms, such as a normal release preparation, a delayed release preparation, a sustained release preparation, or a controlled release preparation.

In some examples, the drug is an enteric preparation, and the preparation may control main release of active ingredients in the intestine. The enteric preparation may be divided according to physical forms, such as an enteric tablet, an enteric capsule, an enteric granule and an enteric pellet; and the enteric preparation may be divided according to specific release sites of the active ingredients, such as a duodenum enteric preparation, a jejunum enteric preparation, an ileum enteric preparation, a cecum enteric preparation, a colon enteric preparation or a rectum enteric preparation.

In some examples, the drug further comprises a pharmaceutically applicable excipient.

In some examples, the food is a dietary supplement or a food for special medical use.

In some examples, the food may be fed by different methods, such as oral feeding, nasal feeding, injection, inhalation, and the like.

In some examples, the food further comprises an additive suitable for food. In some examples, the additive may be a preservative, a colorant, a flavoring agent, a spice, an anti staling agent, an antioxidant, an emulsifier, a thickening agent, a carbohydrate, a fat, a vitamin, an amino acid, a trace element, a protein, and the like.

In some examples, a daily consumption of the lysozyme may be 0.5 g to 20 g.

In some examples, the drug may be administrated during the non-sleeping time at a dosage frequency of once every 1 hour to every 24 hours, such as being administrated once every 1 hour, every 2 hours, every 4 hours, every 6 hours, every 8 hours, every 12 hours, or every 24 hours.

The present disclosure has the beneficial effects as follows.
1. The lysozyme or the combination containing lysozyme of the present disclosure can effectively inhibit cell damage caused by the novel coronavirus, inhibit inflammatory reaction caused by the novel coronavirus, accelerate elimination of the novel coronavirus *in vivo,* shorten detoxification time, and accelerate the speed of turning negative in the novel coronavirus nucleic acid detection *in vivo* (comprising the digestive tract specimen), and can effectively prevent and reduce spread of the novel coronavirus, and reduce an infection rate of the novel coronavirus and a severe case rate of the COVID-19 novel coronavirus pneumonia.
2. The lysozyme or the combination containing lysozyme of the present disclosure can improve airway obstruction and decrease expiratory volume caused by pulmonary lesion of a patient suffering from the novel coronavirus infection, and has a good therapeutic effect on high viral load and respiratory distress of a patient suffering from COVID-19 novel coronavirus pneumonia.
3. Studies show that the patient suffering from the novel coronavirus infection with symptoms in the digestive system are more likely to go through a severe case or a prolonged course of illness; the lysozyme or the combination containing lysozyme of the present disclosure can effectively relieve the symptoms in the digestive system of the patient, improve intestinal barrier function, accelerate the speed of turning negative of the digestive tract specimen of the patient in the novel coronavirus nucleic acid detection, shorten the course of illness of the patient, have a good therapeutic effect on the novel coronavirus infection of the digestive system, and reduce the severe case rate of the patient with the symptoms in the digestive system.
4. The lysozyme or the combination containing lysozyme of the present disclosure has a high safety, and may be used as a preventive drug or an auxiliary preventive food, which can protect cells and organisms from virus attack and damage in an incubation period of the novel coronavirus, inhibit replication of the novel coronavirus *in vivo,* and reduce an infection rate of the novel coronavirus; and the lysozyme or the combination containing lysozyme is helpful for the patient suffering from COVID-19 novel coronavirus pneumonia with atypical symptoms (especially the patient who has not been confirmed yet, such as the patient with latent infection of digestive system with novel coronavirus) to get treatment in time, and reduce the spread risk of virus of the asymptomatic infected patient.

### DETAILED DESCRIPTION

The present disclosure is described in detail hereinafter with reference to the specific implementations. It is to be understood that the contents of the specific implementations are illustrative, and are not restrictive, which means that the specific implementations do not restrict the contents of the present disclosure in any way.

### Definition:

"Lysozyme": the lysozyme of the present disclosure may be lysozyme from animals, plants, and microorganisms, or a recombinant of natural lysozyme. For example, the lysozyme may be a chicken egg white lysozyme, a human lysozyme, a recombinant human lysozyme, a bacteriophage lysozyme, and the like. The lysozyme in the present disclosure includes a medicinal salt of the lysozyme, such as a hydrochloride, a chloride, a sulfate, or an amino acid salt. Lysozyme was first discovered by Fleming as an endogenous enzyme widely existing in organisms. Lysozyme has been approved as a food or a drug worldwide. Lysozyme has been Generally Recognized As Safe(GRAS) in the United States. Lysozyme is allowed to be used as a food additive by WHO, many European countries, Japan, and China. Lysozyme is approved for medicinal use in China, Japan, Singapore, and other countries. At present, lysozyme is known to have a strong antiviral ability against a herpes virus.

"Novel coronavirus", which is namely novel coronavirus 2019, is a virus found in 2019, and belongs to a new virus in the coronaviridae family. In February 2020, the World Health Organization named the virus COVID-19. The COVID-19 in the present disclosure refers to the novel coronavirus 2019.

"COVID-19 novel coronavirus pneumonia" is a pneumonia caused by infection with the novel coronavirus. Pathological features, clinical manifestations, and diagnostic criteria of the COVID-19 novel coronavirus pneumonia are described in detail in the "Diagnosis and Treatment Scheme for Novel Coronavirus Pneumonia" issued by the National Health Commission of the PRC. Many features of COVID-19 novel coronavirus pneumonia are obviously different from those of bacterial pneumonia, viral pneumonia, and atypical pneumonia(severe acute respiratory syndrome, SARS).

Enteric preparation: the enteric preparation refers to a preparation that does not release or hardly releases drugs in stomach, but releases most or all of the drugs in certain parts of intestine after entering the intestine. The intestine of a human body comprises small intestine and large intestine, wherein the small intestine is further divided into duodenum, jejunum and ileum, and the large intestine is further divided into cecum, colon and rectum. Different parts of gastrointestine have different pH values, for example, pH value in the stomach is about 1 to 3, pH value in the small intestine is about 4 to 7, and pH value in the large intestine is about 7 to 8. By using a pH-dependent degradable material as an excipient, a preparation releasing drugs in specific parts of the gastrointestine may be obtained, such as a small intestine enteric preparation or a large intestine enteric preparation. Specifically, the preparation may comprise a duodenum enteric preparation, a jejunum enteric preparation, an ileum enteric preparation, a cecum enteric preparation, a colon enteric preparation or a rectum enteric preparation.

The present disclosure is further illustrated hereinafter with the embodiments, but the embodiments do not restrict the solution and effect of the present disclosure.

### Example 1: application of lysozyme in patients suffering from COVID-19 novel coronavirus pneumonia and high-risk group of infection

During the COVID-19 epidemic in China, our company donated drugs worth more than 2 Million Yuan to Hubei Province twice, comprising lysozyme buccal tablets, lysozyme enteric tablets, and other antibacterial drugs. These drugs played a positive role in anti COVID-19 epidemic. After that, many patients volunteered to take lysozyme products of our company.

Nine newly-confirmed patients suffering from the COVID-19 novel coronavirus pneumonia took the lysozyme buccal tablets and/or the lysozyme enteric tablets, with a dosage range of 0.5 g to 2 g per day, and basically insisted on taking the tablets every day. As a result, only one patient became a severe case, and the other eight patients were all mild cases and then recovered. Upon calculation, a severe case conversion rate was 11.1%.

Three persons who lived and traveled in high-risk areas during the epidemic insisted on administrating the lysozyme buccal tablets and/or the lysozyme enteric tablets every day, with a dosage range of 1 g to 1.5 g every day, and when they returned to work, nucleic acid detections showed no novel coronavirus infection was found in the three persons. The infection rate was 0%.

According to the report of" the Epidemiological Characteristics of an Outbreak of 2019 Novel Coronavirus Diseases (COVID-19) in China ", a proportion of severe patients and critical patients in the people with novel coronavirus pneumonia has reached up to 18.5%, and a death rate was even close to 50% after these patients converted into severe cases and critical cases. By comparison, it was found that administration of a lysozyme preparation could greatly reduce the severe case rate, and then reduce the death rate, thus having a very positive effect on preventing and treating COVID-19 epidemic; in addition, the lysozyme preparation also played a role in preventing infection with novel coronavirus for high-risk group of novel coronavirus infection, which did be of great value for controlling spread and recurrence of the epidemic.

### Example 2: in vitro test of lysozyme for anti novel coronavirus

The effect of lysozyme in anti COVID-19 novel coronavirus was researched with African green monkey kidney cells.

Drug: lysozyme and monoammonium glycyrrhizinate.

Cell: African green monkey kidney cell (Vero E6 cell).

Virus: COVID-19 preserved by Technology Center of Guangzhou Customs, with a titer of 100TCID₅₀.

Culture: 100 µL of Vero E6 cells with a concentration of 2×10⁵ cells/mL were added into each well of a sterile 96-well culture plate, and cultured at 37°C for 24 hours. The culture plates were divided into a blank control group, a virus control group, a low-dose lysozyme group, a medium-dose lysozyme group, a high-dose lysozyme group, a low-dose monoammonium glycyrrhizinate group, and a high-dose monoammonium glycyrrhizinate group according to wells, with 3 wells in each group. Except the blank control group, 100TCID₅₀ virus solution was added into each group by 100 µL/well, and after adsorption in 5% CO₂ incubator at 37°C for 2 hours, the cell culture solution in the culture plate was discarded.

Addition of drug: a drug solution was added into wells of the following groups according to dosages: the low-dose lysozyme group (500 µg/mL), the medium-dose lysozyme group (1,000 µg/mL), the high-dose lysozyme group (2,000 µg/mL), the low-dose monoammonium glycyrrhizinate group (1,000 µg/mL), and the high-dose monoammonium glycyrrhizinate group (2,000 µg/mL), with 100 µL in each well. Then incubation was performed at 37°C for 4 days.

Observation of cytopathic effect: the cytopathic effect (CPE) was observed under an optical microscope after incubation. The degree of the cytopathic effect was recorded according to the following six standards: "-" referred to no cytopathic effect, "+" referred to less than 10% of cytopathic effect, "+" referred to about 25% of cytopathic effect, "++" referred to about 50% of cytopathic effect, "+++" referred to about 75% of cytopathic effect, and "++++" referred to more than 75% of cytopathic effect. OD value of each well was determined by a CCK8 kit and a microplate reader, and the inhibition rate of drug of each group on the cytopathic effect caused by the virus was calculated. Inhibition rate = (As-Av)/(Ac-Av), wherein As was the OD value of each drug group; Av was the OD value of the virus control group; and Ac was the OD value of the blank control group. The higher the inhibition rate, the better the effect.

Results: main results are listed in Table 1. The test results show that the lysozyme may significantly inhibit the cytopathic effect caused by the novel coronavirus. The salt of glycyrrhizic acid has a weak inhibition effect on the novel coronavirus.

**Table 1: inhibition rate of lysozyme on cytopathic effect caused by novel coronavirus**

| Drug (concentration) | Inhibition rate (%) |
|---|---|
| Lysozyme (500 µg/mL) | 15.00±8.66 |
| Lysozyme (1,000 µg/mL) | 46.67±15.28 |
| Lysozyme (2,000 µg/mL) | 78.33±2.89 |
| Monoammonium glycyrrhizinate (1,000 µg/mL) | 23.35±5.43 |
| Monoammonium glycyrrhizinate (2,000 µg/mL) | 29.42±7.16 |

### Example 3: in vitro test of combination containing lysozyme for anti novel coronavirus and inflammation caused by novel coronavirus

Inhibition effects of the combination containing lysozyme on the cytopathic effect caused by the novel coronavirus and on inflammation caused by the novel coronavirus were researched.

The drug, the cell and the virus were all the same as those in Example 2.

Grouping of culture plates and addition amounts of the drug were: a blank control group, a virus control group, a lysozyme group (350 µg/mL), a lysozyme+monoammonium glycyrrhizinate group I (100 µg/mL+1 µg/mL), a lysozyme+monoammonium glycyrrhizinate group II (100 µg/mL+5 µg/mL), a lysozyme+monoammonium glycyrrhizinate group III (100 µg/mL+10 µg/mL), and a lysozyme+monoammonium glycyrrhizinate group IV (100 µg/mL+50 µg/mL).

The method for observing the cytopathic effect was the same as that in Example 2. Test results are shown in Table 2. In addition, cells without the cytopathic effect were collected, RNA was extracted, and relative expression levels of TNF-α, IL-6, and other inflammatory factors were determined by quantitative-PCR. Test results are shown in Table 3.

**Table 2: inhibition effect of combination containing lysozyme on cytopathic effect caused by novel coronavirus**

| Drug (concentration) | Inhibition rate (%) |
|---|---|
| Lysozyme (350 µg/mL) | 6.67±1.56 |
| Lysozyme+monoammonium glycyrrhizinate (100 µg/mL+1 µg/mL) | 38.20±8.11## |
| Lysozyme+monoammonium glycyrrhizinate (100 µg/mL+5 µg/mL) | 49.32±9.45## |
| Lysozyme+monoammonium glycyrrhizinate (100 µg/mL+10 µg/mL) | 23.68±4.31## |
| Lysozyme+monoammonium glycyrrhizinate (100 µg/mL+50 µg/mL) | 8.20±2.27 |

| | |
|---|---|
| Note: each drug combination group compared with the lysozyme group, (#) means P < 0.05 , and (##) means P < 0.01. | |

**Table 3: effect of combination containing lysozyme on expression of cell inflammatory factor induced by novel coronavirus**

| Group | TNF-α mRNA | IL-6 mRNA |
|---|---|---|
| | Relative expression quantity | Relative expression quantity |
| Blank control group | 1.01±0.06 | 1.08±0.07 |
| Virus control group | 3.61±0.29 | 7.68±0.50 |
| Lysozyme group (350 µg/mL) | 3.12±0.26^{∗} | 6.78±0.30^{∗∗} |
| Lysozyme+monoammonium glycyrrhizinate | 2.25±0.36^{∗∗}## | 4.30±0.21^{∗∗}## |
| group I (100 µg/mL+1 µg/mL) | | |
| Lysozyme+monoammonium glycyrrhizinate group II (100 µg/mL+5 µg/mL) | 2.13±0.14^{∗∗}## | 3.47±0.25^{∗∗}## |
| Lysozyme+monoammonium glycyrrhizinate group III (100 µg/mL+10 µg/mL) | 2.65±0.26^{∗∗}# | 5.74±0.31^{∗∗}## |
| Lysozyme+monoammonium glycyrrhizinate group IV (100 µg/mL+50 µg/mL) | 3.15±0.10 | 6.45±0.43^{∗∗} |

| | | |
|---|---|---|
| Note: each drug group compared with the virus control group, (*) means P < 0.05 and (**) means P < 0.01. Each drug combination group compared with the lysozyme group, (#) means P < 0.05 and (##) means P < 0.01. | | |

It can be seen from Table 2 and Table 3 that lysozyme and the drug combination containing lysozyme both have a good inhibition effect on the cytopathic effect caused by the novel coronavirus, and can significantly reduce the increased cell inflammatory factors caused by the novel coronavirus. Compared with the lysozyme alone, the effect of the drug combination is obviously better, which shows that the inhibition rate is significantly improved, the level of inflammatory factors is obviously reduced, and the dosage of drug is greatly reduced. It is suggested that there is a strong synergistic effect between the salt of glycyrrhizic acid and lysozyme, especially when the dosage of the salt of glycyrrhizic acid is small, the synergistic effect is better.

### Example 4: effects of lysozyme and combination of lysozyme on airway resistance and lung function of guinea pig inhaling smoke

A smoked model is a classic animal modeling method of chronic obstructive pulmonary disease. After inhaling smoke for a long time, the animal may suffer from increase of mucus secretion, airway obstruction, and decrease of lung function. The effect of lysozyme on the airway obstruction was evaluated by the modeling method.

### 1. Test drugs

Lysozyme, monoammonium glycyrrhizinate and monopotassium glycyrrhizinate

### 2. Animals and division

Male Hartley guinea pigs with weights ranging from 400 g to 500 g were randomly divided into a blank group, a model group, a lysozyme oral administration group (200 mg/kg), a lysozyme inhalation group (2 mg/kg), a lysozyme+monoammonium glycyrrhizinate inhalation group (2 mg/kg+0.1 mg/kg), a lysozyme+monopotassium glycyrrhizinate oral administration group I (200 mg/kg+20 mg/kg), and a lysozyme+monopotassium glycyrrhizinate oral administration group II (200 mg/kg+40 mg/kg), with eight animals in each group.

### 3. Modeling

The animals in each group except the blank group were exposed to cigarette smoke by using a smoke exposure system inhaled only through nose and mouth, so that the animals inhaled smoke of five cigarettes within 40 minutes every day, for 5 days every week, and for 12 consecutive weeks.

### 4. Drug preparation and administration

Lysozyme and monoammonium glycyrrhizinate used for inhalation administration were respectively crushed into powder with a particle size less than 10 µm. Then lysozyme and monopotassium glycyrrhizinate used for oral administration were respectively dissolved in normal saline.

The animals in each group except the blank group and the model group were administrated once every day for 4 consecutive weeks from the 8^{th} week after modeling. The inhalation groups were administrated by a mouth and nose inhalation exposure system, while the oral administration groups were administrated by oral gavage. The blank group and the model group were administrated by oral gavage with normal saline every day.

### 5. Test

Airway resistance measurement and the measurement of force expiratory volume in 100ms(millisecond) were performed after administration.

The animals were placed in a double chamber plethysmography system, and a specific airway resistance (sRaw) was measured after the animals were quiet for 10 minutes. The animals were anesthetized by intramuscular injection of ketamine, then a catheter was inserted into the trachea, and the force expiratory volume in 100ms (FEV 100) was measured by a lung function detection system.

### 6. Test results and evaluation

Results of the airway resistance measurement and the measurement of force expiratory volume in 100ms of the animals are shown in Table 4.

**Table 4: effects of lysozyme and combination containing lysozyme on airway resistance and force expiratory volume in 100ms of animals**

| Group | Airway resistance (cmH₂O•s) | Force expiratory volume in 100ms (mL) |
|---|---|---|
| Blank group | 1.43±0.24 | 11.86±1.24 |
| Model group | 4.72±0.38 | 4.18±1.26 |
| Lysozyme oral administration group | 3.79±0.30^{∗∗} | 5.79±2.03 |
| Lysozyme inhalation group | 3.10±0.14^{∗∗} | 7.68±1.36^{∗∗} |
| Lysozyme+monoammonium glycyrrhizinate inhalation group | 2.52±0.22^{∗∗}&& | 9.04±0.84^{∗∗} |
| Lysozyme+monopotassium glycyrrhizinate oral administration group I | 2.78±0.17^{∗∗}## | 8.05±1.56^{∗∗}# |
| Lysozyme+monopotassium glycyrrhizinate oral administration group II | 3.47±0.28^{∗∗} | 5.89±1.01 |

| | | |
|---|---|---|
| Note: each administration group compared with the model group, (*) means P < 0.05 and (**) means P < 0.01. The oral administration combination group compared with the lysozyme oral administration group, (#) means P < 0.05 and (##) means P < 0.01. The inhalation combination group compared with the lysozyme inhalation group, (&) means p < 0.05 and (&&) means p < 0.01. | | |

In the test, long-term inhalation of smoke causes a significant increase in lung airway resistance and a significant decrease in lung function (reduction in expiratory volume) of the animals. It can be seen from the test results that each administration group reduces the lung airway resistance and increases the expiratory volume to varying degrees. By comparing the drug combination group with the lysozyme group alone, it is found that the glycyrrhizic acid compound enhances the effect of lysozyme, especially when the dosage of the glycyrrhizic acid compound is small, the effect is significantly enhanced.

### Example 5: protective effects of lysozyme and combination containing lysozyme on intestinal barrier dysfunction of mice induced by lipopolysaccharide

Lipopolysaccharide could cause intestinal dysfunction of animals. The protective effects of the lysozyme and the combination containing lysozyme on the intestinal barrier dysfunction of mice induced by the lipopolysaccharide were researched in the test.

Test drugs: lysozyme, monoammonium glycyrrhizinate, and dipotassium glycyrrhizinate.

Test animals: male C57BL/6 mice with a cleaning degree, and with a weight ranging from 20 g to 25 g.

Grouping and administration of animals: the animals were randomly divided into a blank group, a model group, a lysozyme group (100 mg/kg), a lysozyme+monoammonium glycyrrhizinate group A (100 mg/kg+2 mg/kg), a lysozyme+monoammonium glycyrrhizinate group B (100 mg/kg+10 mg/kg), a lysozyme+monoammonium glycyrrhizinate group C (100 mg/kg+20 mg/kg), and a lysozyme+dipotassium glycyrrhizinate group (100 mg/kg+2 mg/kg), with eight animals in each group. Each drug group was administrated by oral gavage according to the dosage every day, while the blank group and the model group were administrated with normal saline. The administration was continuously performed for 30 days.

Modeling: after the administration, the animals of each group except the blank group were intraperitoneally injected with 15 mg/kg lipopolysaccharide, and the blank group was injected with the normal saline. The animals were fasted for 12 hours after injection.

Measurement of intestinal permeability: animals of each group were administrated by oral gavage with 600 mg/kg FITC-glucan (dissolved in a phosphate buffer), and blood was taken from an eyeball after 4 hours. The content of the FITC-glucan in serum was measured by fluorescence spectrometry (with an excitation wavelength of 480 nm and an emission wavelength of 520 nm). Results are shown in Table 5.

Determination of the expression of intestinal tissue tight junction protein: after the animals were killed, the jejunum tissue sample was taken to extract RNA, and relative expression levels of occludin protein and Claudin-1 protein in the tissue were detected by quantitative PCR. Results are shown in Table 6.

**Table 5: effects of lysozyme and combination containing lysozyme on intestinal permeability**

| Group | Content of FITC-glucan (µg/mL) |
|---|---|
| Blank group | 9.40±1.45 |
| Model group | 35.06±5.75 |
| Lysozyme group (100 mg/kg) | 27.53±3.21^{∗∗} |
| Lysozyme+monoammonium glycyrrhizinate group A (100 mg/kg+2 mg/kg) | 15.31±2.08^{∗∗}## |
| Lysozyme+monoammonium glycyrrhizinate group B (100 mg/kg+10 mg/kg) | 20.25±2.44^{∗∗}## |
| Lysozyme+monoammonium glycyrrhizinate group C (100 mg/kg+20 mg/kg) | 23.89±2.52^{∗∗} |
| Lysozyme+dipotassium glycyrrhizinate group (100 mg/kg+2 mg/kg) | 13.17±1.14^{∗∗}## |

| | |
|---|---|
| Note: each drug group compared with the model group, (^{∗}) means P < 0.05 and (^{∗∗}) means P < 0.01 Each drug combination group compared with the lysozyme group, (#) means P < 0.05 and (##) means P < 0.01. | |

**Table 6: effects of lysozyme and combination containing lysozyme on expression of intestinal tissue tight junction protein**

| Group | Occludin mRNA | Claudin-1 mRNA |
|---|---|---|
| | Relative expression quantity | Relative expression quantity |
| Blank group | 1.04±0.12 | 1.07±0.11 |
| Model group | 0.54±0.09 | 0.73±0.13 |
| Lysozyme group (100 mg/kg) | 0.69±0.07^{∗∗} | 0.79±0.16 |
| Lysozyme+monoammonium glycyrrhizinate group A (100 mg/kg+2 mg/kg) | 1.22±0.24^{∗∗}## | 1.30±0.19^{∗∗}## |
| Lysozyme+monoammonium glycyrrhizinate group B (100 mg/kg+10 mg/kg) | 0.93±0.16^{∗∗}## | 1.11±0.15^{∗∗}## |
| Lysozyme+monoammonium glycyrrhizinate group C (100 mg/kg+20 mg/kg) | 0.67±0.10^{∗} | 0.83±0.17 |
| Lysozyme+dipotassium glycyrrhizinate group (100 mg/kg+2 mg/kg) | 1.07±0.07^{∗∗}## | 1.00±0.16^{∗∗}# |

| | | |
|---|---|---|
| Note: each drug group compared with the model group, (^{∗}) means P < 0.05 and (^{∗∗}) means P < 0.01. Each drug combination group compared with the lysozyme group, (#) means P < 0.05 and (##) means P < 0.01. | | |

The protective effects of lysozyme and the combination containing lysozyme on the intestinal barrier dysfunction of mice induced by the lipopolysaccharide are researched in the test. After injection of the lipopolysaccharide, it is found that the model group has a significantly increased content of serum glucan compared with the blank group, which indicates that a large amount of glucan enters blood through intestine, that is, the intestinal permeability is increased. The expressions of two tight junction proteins in the intestine are decreased in the model group, which further indicates that the intestinal barrier function is impaired. It is found through the test that lysozyme and the combination containing lysozyme can significantly reduce the increased intestinal permeability induced by lipopolysaccharide, and significantly increase the expression of the tight junction protein in the intestinal tissue, thus having an obvious protective effect on the intestinal barrier dysfunction. It is also found that the effect of the drug combination group is obviously better than that of the lysozyme alone.

### Example 6: application of lysozyme in patients suffering from intestine infection with novel coronavirus

Respiratory tract specimens of 10 patients suffering from COVID-19 in convalescence were negative in novel coronavirus nucleic acid detection, the patients had no clinical respiratory tract symptoms, but stool specimens of the patients were positive in the novel coronavirus nucleic acid detection, so that the patients belonged to intestinal infection and were subject to medical isolation. Among them, 7 patients had digestive tract symptoms such as diarrhea, abdominal pain and flatulence, and 3 patients had no obvious digestive tract symptoms. After administrating 4 g to 6 g of lysozyme enteric tablets as described in Example 1 every day, compared with the same batch of other isolated patients whose respiratory tract specimens were negative in the novel coronavirus nucleic acid detection but the stool specimens were positive in the novel coronavirus nucleic acid detection (comprising patients with digestive tract symptoms), it was observed that the digestive tract symptoms of the patients who were administrated with the lysozyme disappeared faster, and intestine specimens turned negative in the novel coronavirus nucleic acid detection more than 3 days earlier.

It is found through comparative research that the lysozyme preparation can accelerate elimination of the novel coronavirus in the patients, shorten the detoxification time of the virus, improve the digestive tract symptoms caused by the novel coronavirus infection in the intestine, and accelerate the speed of turning negative of the digestive tract specimens of the patients in the novel coronavirus nucleic acid detection.

In conclusion, lysozyme or the combination containing lysozyme of the present disclosure can effectively inhibit the cell damage caused by the novel coronavirus, inhibit expression of the inflammatory factors caused by the novel coronavirus, accelerate elimination of the virus *in vivo,* shorten the detoxification time, and accelerate the speed of turning negative in the novel coronavirus nucleic acid detection; and lysozyme or the combination containing lysozyme can relieve the digestive tract symptoms, improve intestinal barrier dysfunction, improve pulmonary airway obstruction, increase expiratory volume, and reduce the infection rate of the novel coronavirus and the conversion rate to severe case COVID-19. According to current clinical knowledge, the novel coronavirus infection may lead to many diseases, the patients have a high viral load in the early stage, an obvious respiratory distress symptom in the middle and late stage in the respiratory system, severe cytokine storm syndrome in the immune system, intestinal barrier dysfunction in the digestive system (which may result in persistent and secondary infection, difficulty in recovery, and increased infectivity), and a high death rate in severe cases. Therefore, lysozyme and the combination containing lysozyme of the present disclosure are expected to prevent and treat the novel coronavirus infection and the novel coronavirus infection and related disease or symptom thereof in various ways. Moreover, due to the good safety of lysozyme and the combination containing lysozyme, the dosage can be increased, so that lysozyme and the combination containing lysozyme are expected to become a better choice for preventing or treating the novel coronavirus infection-related disease or symptom. Meanwhile, with the deepening of research, it is found that the novel coronavirus is active and exists for a long time in the digestive tract, which may appear earlier than clinical symptoms. The digestive tract specimens are still positive even after the respiratory tract specimens turn negative in the novel coronavirus nucleic acid detection. Patients with atypical symptoms or asymptomatic infected patients are constantly found, which further reflects the complexity of the COVID-19 epidemic, so that prevention and control cannot be slackened. The novel coronavirus has a high infection rate, a long incubation period, a strong concealment, a short continuous interval and a long virus detoxification time. Lysozyme and the combination containing lysozyme of the present disclosure are expected to provide timely treatment for the patients suffering from the novel coronavirus infection (comprising infection in the digestive system) with atypical clinical symptoms, and can shorten the course of illness in patients with digestive tract symptoms, and effectively prevent spread of the novel coronavirus, thus having a good application prospect.

## Claims

1. An application of lysozyme or a combination containing lysozyme in preparation of a drug for anti a novel coronavirus infection or a drug for preventing/treating a novel coronavirus infection-related disease or symptom.

2. The application according to claim 1, wherein the novel coronavirus infection is a novel coronavirus infection of respiratory system and/or digestive system.

3. The application according to claim 1, wherein the novel coronavirus infection-related disease or symptom is at least one selected from the group consisting of disease or symptom of respiratory system, disease or symptom of immune system, and disease or symptom of digestive system.

4. The application according to claim 3, wherein the disease or symptom of immune system is selected from fever or cytokine storm syndrome.

5. The application according to claim 3, wherein the disease or symptom of digestive system is selected from intestinal barrier dysfunction or digestive dysfunction.

6. The application according to claim 3, wherein the disease or symptom of respiratory system is selected from increased airway resistance, decreased expiratory volume, pneumonia or acute respiratory distress syndrome.

7. The application according to claim 1, wherein the novel coronavirus infection-related disease or symptom is COVID-19.

8. The application according to any one of claim 1 or 7, wherein the combination further comprises glycyrrhizic acid or a salt of the glycyrrhizic acid.

9. The application according to claim 8, wherein a weight ratio of the lysozyme to the glycyrrhizic acid or the salt of the glycyrrhizic acid in the combination is 100: 1 to 1: 100, preferably 100: 1 to 2: 1, more preferably 100: 1 to 5: 1, and further preferably 100: 1 to 10: 1.

10. The application according to any one of claim 1, 7, 8 or 9, wherein the combination further comprises other ingredients capable of being used for preventing or treating the novel coronavirus infection-related disease or symptom.

11. The application according to claim 10, wherein:
the other ingredients capable of being used for preventing or treating the novel coronavirus infection-related disease or symptom comprise other ingredients capable of being used for preventing or treating COVID-19 and/or other ingredients capable of being used for preventing or treating the disease or symptom of digestive system.

12. The application according to claim 11, wherein:
the other ingredients capable of being used for preventing or treating COVID-19 are at least one selected from the group consisting of interferon, oseltamivir or salt thereof, lopinavir, ritonavir, favipiravir, ribavirin, remdesivir, chloroquine or salt thereof, hydroxychloroquine or salt thereof, arbidol or salt thereof, interleukin inhibitors, tumor necrosis factor inhibitors, janus kinase inhibitors, glucocorticoid or protease inhibitors, and intestinal microecological regulators; and
the other ingredients capable of being used for preventing or treating the disease or symptom of digestive system are at least one selected from the group consisting of intestinal microecological regulators, gastrointestinal mucosal protective agents, antiemetic, antidiarrheal, gastrointestinal excitomotors, antispasmodic, antiulcere agents, and digestant.

13. The application according to claim 8 or 11, wherein at least one of the lysozyme, the glycyrrhizic acid or the salt of the glycyrrhizic acid, the other ingredients capable of being used for preventing or treating the COVID-19, and the other ingredients capable of being used for preventing or treating the disease or symptom of digestive system in the combination exists in different drugs respectively, or co-exists in the same drug.

14. The application according to claim 1 or 7, wherein dosage forms of the drug comprise an oral dosage form, an injection dosage form, an inhalation dosage form, and a cavity dosage form.

15. The application according to claim 1 or 7, wherein a release form of the drug is normal release, delayed release, sustained release, or controlled release.

16. The application according to claim 1 or 7, wherein the drug further comprises a pharmaceutically acceptable excipient.

17. A drug, wherein the drug comprises lysozyme, and the drug is used for anti novel coronavirus infection or preventing/treating novel coronavirus infection-related disease or symptom.

18. The drug according to claim 17, wherein the novel coronavirus infection is a novel coronavirus infection of respiratory system and/or digestive system.

19. The drug according to claim 17, wherein the novel coronavirus infection-related disease or symptom is at least one selected from the group consisting of diseases or symptoms of immune system, diseases or symptoms of respiratory system, and diseases or symptoms of digestive system.

20. The drug according to claim 17, wherein the novel coronavirus infection-related disease or symptom is COVID-19.

21. The drug according to claim 17 or 20, wherein the drug further comprises glycyrrhizic acid or a salt of the glycyrrhizic acid.

22. The drug according to claim 21, wherein a weight ratio of the lysozyme to the glycyrrhizic acid or the salt of the glycyrrhizic acid in the drug is 100: 1 to 1: 100, preferably 100: 1 to 2: 1, more preferably 100: 1 to 5: 1, and further preferably 100: 1 to 10: 1.

23. The drug according to any one of claim 17, 20, 21 or 22, wherein the drug further comprises other ingredients capable of being used for preventing or treating the novel coronavirus infection-related disease or symptom.

24. The drug according to claim 23, wherein the other ingredients capable of being used for preventing or treating the novel coronavirus infection-related disease or symptom comprise other ingredients capable of being used for preventing or treating the COVID-19 and/or other ingredients capable of being used for preventing or treating the disease or symptom of digestive system.

25. The drug according to claim 24, wherein:
the other ingredients capable of being used for preventing or treating the COVID-19 are at least one selected from the group consiting of interferon, oseltamivir or salt thereof, lopinavir, ritonavir, favipiravir, ribavirin, remdesivir, chloroquine or salt thereof, hydroxychloroquine or salt thereof, arbidol or salt thereof, interleukin inhibitors, tumor necrosis factor inhibitors, janus kinase inhibitors, glucocorticoid or protease inhibitors, and intestinal microecological regulators; and
the other ingredients capable of being used for preventing or treating the disease or symptom of digestive system are at least one selected from the group consisting of intestinal microecological regulators, gastrointestinal mucosal protective agents, antiemetic, antidiarrheal, gastrointestinal excitomotors, antispasmodic, antiulcer agents, and digestant.

26. The drug according to claim 21 or 24, wherein at least one of the lysozyme, the glycyrrhizic acid or the salt of the glycyrrhizic acid, the other ingredients capable of being used for preventing or treating COVID-19, and the other ingredients capable of being used for preventing or treating the disease or symptom of digestive system in the combination exists in different drugs respectively, or co-exists in the same drug.

27. The drug according to claim 17 or 20, wherein dosage forms of the drug comprise an oral dosage form, an injection dosage form, an inhalation dosage form, and a cavity dosage form.

28. The drug according to claim 17 or 20, wherein a release form of the drug is normal release, delayed release, sustained release, or controlled release.

29. The drug according to claim 17 or 20, wherein the drug is an enteric preparation.

30. The drug according to claim 17 or 20, wherein the drug further comprises a pharmaceutically acceptable excipient.

31. A food, wherein the food comprises lysozyme, and the food is used for assisting in anti a novel coronavirus infection and assisting in preventing or treating a novel coronavirus infection-related disease or symptom.

32. The food according to claim 31, wherein the novel coronavirus infection is a novel coronavirus infection of respiratory system and/or digestive system.

33. The food according to claim 31, wherein the novel coronavirus infection-related disease or symptom is COVID-19.

34. The food according to claim 31 or 33, wherein the food is a dietary supplement or a food for special medical use.

35. The food according to claim 31 or 33, wherein the food further comprises glycyrrhizic acid or a salt of the glycyrrhizic acid.

36. The food according to claim 35, wherein a weight ratio of the lysozyme to the glycyrrhizic acid or the salt of the glycyrrhizic acid in the food is 100: 1 to 1: 100, preferably 100: 1 to 2: 1, more preferably 100: 1 to 5: 1, and further preferably 100: 1 to 10: 1.

37. The food according to claim 35, wherein the lysozyme and the glycyrrhizic acid or the salt of the glycyrrhizic acid in the food respectively and independently exist in different foods, or co-exist in the same food.

38. The food according to claim 31 or 33, wherein the food further comprises an additive suitable for food.

39. An application of lysozyme or a combination containing lysozyme in preparation of a food, wherein the food is a food for anti a novel coronavirus infection or a food for assisting in preventing/treating a novel coronavirus infection-related disease or symptom.

40. The application according to claim 39, wherein the novel coronavirus infection is a novel coronavirus infection of respiratory system and/or digestive system.

41. The application according to claim 39, wherein the novel coronavirus infection-related disease or symptom is COVID-19.

42. The application according to claim 39 or 41, wherein the food is a dietary supplement or a food for special medical use.

43. The application according to claim 39 or 41, wherein the food further comprises glycyrrhizic acid or a salt of the glycyrrhizic acid.

44. The application according to claim 43, wherein the lysozyme and the glycyrrhizic acid or the salt of the glycyrrhizic acid in the combination respectively exist in different foods, or co-exist in the same food.

45. The application according to claim 43, wherein a weight ratio of the lysozyme to the glycyrrhizic acid or the salt of the glycyrrhizic acid in the food is 100: 1 to 1: 100, preferably 100: 1 to 2: 1, more preferably 100: 1 to 5: 1, and further preferably 100: 1 to 10: 1.

46. The application according to claim 39, 41 or 43, wherein the food further comprises an additive suitable for food.

47. The application according to claim 46, wherein the additive is at least one selected from the group consisting of preservative, colorant, flavoring agents, spice, antistaling agents, antioxidant, emulsifier, thickening agents, carbohydrate, fat, vitamin, amino acid, trace elements, or protein.

48. A method for anti a novel coronavirus infection or preventing/treating a novel coronavirus infection-related disease or symptom, comprising administering a drug to a subject in need thereof, wherein the drug comprises an effective amount of lysozyme, or the drug comprises an effective amount of a combination comprising lysozyme.

49. The method according to claim 48, wherein the novel coronavirus infection is a novel coronavirus infection of respiratory system and/or digestive system.

50. The method according to claim 48, wherein the novel coronavirus infection-related disease or symptom is at least one selected from the group consisting of diseases or symptoms of immune system, diseases or symptoms of respiratory system, and diseases or symptoms of digestive system.

51. The method according to claim 50, wherein:
the disease or symptom of immune system is selected from fever or cytokine storm syndrome;
the disease or symptom of digestive system is selected from intestinal barrier dysfunction or digestive dysfunction;
the disease or symptom of respiratory system is selected from increased airway resistance, decreased expiratory volume, pneumonia or acute respiratory distress syndrome.

52. The method according to claim 48, wherein the novel coronavirus infection-related disease or symptom is COVID-19.

53. The method according to claim 48, wherein the subject is positive in a novel coronavirus nucleic acid detection.

54. The method according to claim 48 or 52, wherein the combination further comprises glycyrrhizic acid or a salt of the glycyrrhizic acid.

55. The method according to claim 54, wherein a weight ratio of the lysozyme to the glycyrrhizic acid or the salt of the glycyrrhizic acid in the combination is 100: 1 to 1: 100, preferably 100: 1 to 2: 1, more preferably 100: 1 to 5: 1, and further preferably 100: 1 to 10: 1.

56. The method according to any one of claim 48, 52 or 54, wherein the combination further comprises other ingredients capable of being used for preventing or treating the novel coronavirus infection-related disease or symptom.

57. The method according to claim 56, wherein the other ingredients capable of being used for preventing or treating the novel coronavirus infection-related disease or symptom comprise other ingredients capable of being used for preventing or treating COVID-19 and/or other ingredients capable of being used for preventing or treating the disease or symptom of digestive system.

58. The method according to claim 57, wherein:
the other ingredients capable of being used for preventing or treating COVID-19 are at least one selected from the group consisting of interferon, oseltamivir or salt thereof, lopinavir, ritonavir, favipiravir, ribavirin, remdesivir, chloroquine or salt thereof, hydroxychloroquine or salt thereof, arbidol or salt thereof, interleukin inhibitors, tumor necrosis factor inhibitors, janus kinase inhibitors, glucocorticoid or protease inhibitors, and intestinal microecological regulators; and
the other ingredients capable of being used for preventing or treating the disease or symptom of digestive system are at least one selected from the group consisting of intestinal microecological regulators, gastrointestinal mucosal protective agents, antiemetic, antidiarrheal, gastrointestinal excitomotors, antispasmodic, antiulcer agents, and digestant.

59. The method according to claim 54 or 57, wherein at least one of the lysozyme, the glycyrrhizic acid or the salt of the glycyrrhizic acid, the other ingredients capable of being used for preventing or treating COVID-19, and the other ingredients capable of being used for preventing or treating the disease or symptom of digestive system in the combination exists in different drugs respectively, or co-exists in the same drug.

60. The method according to claim 48 or 52, wherein an administration method of the drug is oral administration, injection, inhalation, or cavity administration.

61. The method according to claim 48 or 52, wherein a release form of the drug is normal release, delayed release, sustained release, or controlled release.

62. The method according to claim 48 or 52, wherein a daily consumption of the lysozyme is 0.5 g to 20 g.

63. The method according to claim 48 or 52, wherein the drug is administrated during the non-sleeping time at a dosage frequency of once every 1 hour to every 24 hours, and is preferably administrated once every 1 hour, every 2 hours, every 4 hours, every 6 hours, every 8 hours, every 12 hours, or every 24 hours.

64. A method for assisting in resisting a novel coronavirus infection or assisting in preventing/treating a novel coronavirus infection-related disease or symptom, comprising feeding a food to a subject in need thereof, wherein the food comprises an effective amount of lysozyme, or the food comprises an effective amount of a combination comprising lysozyme.

65. The method according to claim 64, wherein the subject is positive in a novel coronavirus nucleic acid detection.

66. The method according to claim 64, wherein the novel coronavirus infection is a novel coronavirus infection of respiratory system and/or digestive system.

67. The method according to claim 64, wherein the novel coronavirus infection-related disease or symptom is at least one selected from the group consisting of increased airway resistance, decreased expiratory volume, pneumonia, acute respiratory distress syndrome, intestinal barrier dysfunction, digestive dysfunction, and fever or cytokine storm syndrome.

68. The method according to claim 64, wherein the novel coronavirus infection-related disease or symptom is COVID-19.

69. The method according to claim 64 or 68, wherein the food is a dietary supplement or a food for special medical use.

70. The method according to claim 64 or 68, wherein the combination further comprises glycyrrhizic acid or a salt of the glycyrrhizic acid.

71. The method according to claim 70, wherein the lysozyme and the glycyrrhizic acid or the salt of the glycyrrhizic acid in the combination respectively exist in different foods, or the lysozyme and the glycyrrhizic acid or the salt of the glycyrrhizic acid co-exist in a same food.

72. The method according to claim 70, wherein a weight ratio of the lysozyme to the glycyrrhizic acid or the salt of the glycyrrhizic acid in the combination is 100: 1 to 1: 100, preferably 100: 1 to 2: 1, more preferably 100: 1 to 5: 1, and further preferably 100: 1 to 10: 1.

73. The method according to claim 64 or 68, wherein a feeding method of the food is oral feeding, nasal feeding, injection, or inhalation.

74. The method according to claim 64 or 68, wherein a release form of the food is normal release, delayed release, sustained release, or controlled release.

75. The method according to claim 64 or 68, wherein a daily consumption of the lysozyme is 0.5 g to 20 g.

76. The method according to claim 64 or 68, wherein the food is fed during the non-sleeping time at a dosage frequency of once every 1 hour to every 24 hours, and is preferably fed once every 1 hour, every 2 hours, every 4 hours, every 6 hours, every 8 hours, every 12 hours, or every 24 hours.
